## Europäisches Patentamt

## European Patent Office

## Office européen des brevets

(19)

(11) Publication number: **0 008 171**
**B1**

(12)

# EUROPEAN PATENT SPECIFICATION

(45) Date of publication of patent specification: **11.11.81**

(51) Int. Cl.³: **A 61 K 7/06**

(21) Application number: **79301446.5**

(22) Date of filing: **20.07.79**

(54) Hair treatment composition and method.

| | |
|---|---|
| (30) Priority: **21.07.78 GB 3072578** | (73) Proprietor: **UNILEVER PLC**<br>**Unilever House Blackfriars**<br>**London EC4 (GB)** |
| (43) Date of publication of application:<br>**20.02.80 Bulletin 80/4** | (84) **GB**<br>(73) Proprietor: **UNILEVER NV**<br>**Burgemeester 's Jacobplein 1**<br>**NL-3000 DK Rotterdam (NL)** |
| (45) Publication of the grant of the European patent:<br>**11.11.81 Bulletin 81/45** | (84) **BE CH DE FR IT NL SE AT** |
| (84) Designated Contracting States:<br>**AT BE CH DE FR GB IT NL SE** | (72) Inventor: **Mathur, Girish Prasad**<br>**33 Landmark, 175 Carter Road**<br>**Bandra, Bombay 400 040 (IN)**<br>Inventor: **Anavkar, Arun Shantaram**<br>**13 Alice Court, Monghal Lane Behind Mahin P.O.**<br>**Mahim, Bombay 400 016 (IN)**<br>Inventor: **Menon, Kuruvakkat Kochu Govind**<br>**6 Kamani House, Peddar Road**<br>**Bombay 400 026 (IN)** |
| (56) References cited:<br>**DE - A - 2 242 553**<br>**DE - B - 1 017 331**<br>**FR - A - 1 418 905**<br>**GB - A - 1 126 018**<br>**GB - A - 1 408 036** | |
| | (74) Representative: **Tonge, Robert James et al,**<br>**Unilever PLC, Patent Division PO Box 31 Salisbury**<br>**Square House Salisbury Square**<br>**London EC4P 4AN (GB)** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 008 171

## Hair treatment composition and method

The invention relates to novel compositions which are capable of stimulating hair growth, and/or restoring hair growth, and/or reducing hair loss in human beings.

Except for the palms and soles, the entire skin surface of human beings is normally hirsute. Hair grows from the cell divisions of matrix cells in a region known as the bulb. In all hairy animals (with the possible exception of merino sheep and poodle dogs) hair follicles show cyclic activity. In such species as mice and rats all hairs are in the same state of activity and their cyclic changes are synchronised. However, in human beings and in animals such as guinea pigs, the cycle of each follicle occurs independently from those of the neighbouring follicles and show a mosaic pattern. The hair cycle is divided into three stages: anagen (growing or active stage), catagen (regressive stage) and telogen (resting stage). The rate of growth of the hair varies from species to species and, in man, from region to region. During human life hair cycles are reproduced repeatedly. The length of hair cycle also varies from species to species and there are regional variations as well.

Most human body hairs grow to predictable lengths peculiar to the body area and to the individual. Like all hairs they undergo periodic shedding and are replaced by other hairs of the same length. The number of hairs in men and women are nearly the same. Of 90,000—100,000 scalp hairs in humans, irrespective of sex, about 90% are in growing (anagen) stage and about 10% in resting (telogen) stage. Human hair grows at the rate of about 0.3—0.4 mm per day but this is influenced by many factors.

Scalp hair in humans has been valued for its adornment value since antiquity. Alopecia or scalp hair loss in man usually occurs with senility. However, chronic hair losses in both yound males and females have been observed during or after psychological stress, high fever, surgical shock, treatment by antibiotics, drugs (e.g. methotrexate, 6-mercaptopurine, actinomycin D, aminopterin, 5-fluorouracil, cyclophosphamid, etc.), X-ray treatment, after pregnancy or after discontinuation of oral contraceptives, and, even during strict diet control. Although luxuriant growth of hair is normal and natural in most people, many people use hair tonic to promote healthy hair growth and reduce falling hair. Many such hair tonics contain a variety of natural substances, e.g. oil of cade, capsicum extract, cantharidin, jaborbandi leaf extract (contains alkaloid pilocarpine), oils from beries of *Emblica afficinalis Gaertn*, juice of flowers of *Hibiscus rosa sinesis*, umbilical cord and placental extract, extract of tea and coffee, etc. Many chemical compounds like sulphur-containing amino acids, cysteine or thiazolidine-4-carboxylic acid, N-acetyl homocysteine thiolactone, camphor, quinine, inositol, hydrocortisone, pantothenic acid, pantothenol, linoleic acid, arachidonic acid, etc., have all been claimed as useful ingredients for hair tonics.

The biological mechanisms regulating proliferation and differentiation of hair follicular cells are not fully understood and little is known about the cause of alopecia. Our studies suggest that proper balance of cyclic adenosine-3′,5′-monophosphate (cAMP) and cyclic guanosine-3′,5′-monophosphate (cGMP) in hair bulb is one of the important factors in controlling follicular activity. Imbalance of hair bulb cAMP and cGMP can result in increased hair loss, ultimately leading to alopecia. We have now found that topical application of imidazole and its derivatives like histidine, methionine, folic acid and p-aminobenzoic acid (PABA) tend to stimulate hair growth in model test systems. We have also found that topical applications of cocktails of imidazole or its derivatives like histidine, with methionine or PABA, and with methionine + PABA, are effective in stimulating hair growth and in increasing hair diameters.

It has been proposed in British Patent Specification No. 1,408,036 to provide a composition for treating the hair and the scalp by topical administrations. The composition optionally comprises p-amino benzoic acid and a mixture of amino acids which can include up to 0.005% by weight histidine and up to 0.12% by weight methionine.

It has also been proposed in French Patent Specification No. 1,418,905 to employ up to 1% by weight of imidazole in a cosmetic composition and in British Patent Specification No. 1,126,018 to employ a mixture of amino acids, including methionine and histidine, in cosmetic and pharmaceutical compositions.

It has also been proposed in German Patent Specification No. 1,017,331 to employ 0.25% by weight of p-amino benzoic acid in a hair treatment product, and in German Patent Specification No. 2,242,553 up to 0.06% by weight of folic acid in a cosmetic composition.

The object of this invention is to provide novel compositions which stimulate hair growth and/or reduce falling hair. The invention accordingly provides a hair and scalp treatment composition comprising a diazole chosen from imidazole, histidine and histidine monohydrochloride, and mixtures thereof; and a monobasic acid chosen from methionine, p-aminobenzoic acid and mixtures thereof; and folic acid, together with a physiologically acceptable carrier.

The composition can also optionally comprise inositol, zinc or magnesium compounds such as zinc oxide and zinc sulphate or magnesium sulphate and magnesium stearate or mixtures thereof which are believed to further contribute to an improvement in the general health of the hair and scalp.

The amounts of substances, as hereinbefore defined which, when present, can be employed in the composition are as follows:

| | |
|---|---|
| Imidazole | from 0.1 to 5.0%, preferably from 1.0 to 3.0% by weight |
| Histidine, or histidine monohydrochloride | from 0.1 to 10.0%, preferably from 0.2 to 3.0% by weight |
| Folic acid | from 0.1 to 5.0%, preferably from 0.2 to 2.0% by weight |
| Methionine | from 0.1 to 5.0%, preferably from 0.2 to 3.0% by weight |
| p-amino benzoic acid | from 0.1 to 5.0%, preferably from 0.2 to 3.0%, by weight |
| Myo-inositol | from 0.1 to 5.0%, preferably from 0.2 to 3.0% by weight |
| Zinc oxide or sulphate | from 0.1 to 1.5%, preferably from 0.1 to 0.6% by weight |
| Magnesium sulphate or stearate | from 0.1 to 1.5%, preferably from 0.1 to 0.6% by weight |
| Soya- or egg-lecithin | from 0.1 to 5%, preferably from 0.1 to 1% by weight |

The preferred form of histidine, when employed, is the salt 1-histidine monohydrochloride. The preferred form of methionine, when employed, is the isomer dl-methionine.

The composition according to the invention also comprises a physiologically acceptable carrier, the nature of which is unimportant, provided that it is safe in use and that it does not diminish the effectiveness of the active ingredients. Examples of physiologically acceptable carriers are oils, fats, phospholipids such as lecithin, certain fatty acids, and long chain alcohols such as cetyl alcohol, usually in the form of an emulsion or alcoholic suspension or solution. The scope of this invention is however not limited to these examples of carriers.

The composition of the invention may be in any of the physical forms which are suitable for cosmetic application to the scalp, for example hair creams, pourable emulsion hair dressings and alcoholic hair lotions.

The invention will now be described by way of the following examples of hair creams, pourable emulsion hair dressings and alcoholic lotions found useful in stimulating hair growth and/or reducing falling hair in human subjects. The quantities of each quoted ingredient are in parts by weight.

## HAIR CREAM

| | | Example 1 | Example 2 | Example 3 | Example 4 | Example 5 |
|---|---|---|---|---|---|---|
| | 1. Mineral oil | — | 32.000 | — | — | — |
| | 2. Coconunt oil | 40.000 | — | 27.000 | 40.000 | 27.000 |
| | 3. Petroleum jelly | — | 5.000 | 10.000 | — | 10.000 |
| | 4. Beeswax | 2.300 | 2.300 | 2.300 | 2.300 | 2.300 |
| | 5. Stearic acid | 2.225 | 2.225 | 2.225 | 2.225 | 2.225 |
| A | 6. Cetyl alcohol | 0.200 | 0.200 | 0.200 | 0.200 | 0.200 |
| | 7. Nipagin M* | 0.100 | 0.100 | 0.100 | 0.100 | 0.100 |
| | 8. BHA | 0.100 | 0.100 | 0.100 | 0.100 | 0.100 |
| | 9. Lanolin | 0.300 | 0.300 | 0.300 | 0.300 | 0.300 |
| | 10. Egg lecithin | 0.500 | 0.500 | 0.500 | 0.500 | 0.500 |
| | 11. Arlacel-83* | 0.100 | 0.100 | 0.100 | 0.100 | 0.100 |
| B | 12. Magnesium sulphate | 0.800 | 0.600 | 0.800 | 0.800 | 0.800 |
| | 13. Zinc sulphate | 0.400 | 0.300 | 0.400 | 0.400 | 0.400 |

* Trade Mark

**0 008 171**

HAIR CREAM (Continued)

|  | Example 1 | Example 2 | Example 3 | Example 4 | Example 5 |
|---|---|---|---|---|---|
| 14. Imidazole | 3.00 | — | — | — | — |
| 15. Histidine | — | 1.00 | 2.00 | 1.50 | 4.0 |
| 16. Methionine | 1.00 | 3.00 | — | 0.5 | 1.0 |
| C 17. P-amino benzoic acid | 0.50 | 1.00 | 2.00 | 1.5 | — |
| 18. Folic acid | 1.00 | 2.00 | 1.00 | 2.0 | 0.5 |
| 19. Inositol | 0.50 | 2.00 | — | 1.0 | 2.0 |
| 20. NaOH | to pH 6.5—7.5 | to pH 6.5—7.5 | to pH 6.5—7.5 | to pH 6.5—7.5 | to pH 6.5—7.5 |
| D Lime water | 14.625 | 14.625 | 14.625 | 14.625 | 14.625 |
| Perfume | 0.500 | 0.500 | 0.500 | 0.500 | 0.500 |
| Distilled water | to 100 | to 100 | to 100 | to 100 | to 100 |

Method of Preparation of Hair Cream

Step I —Mix the ingredients of A and heat to 50—60°C in a water bath.

II —Dissolve components of part B in 5—10 ml of water.

III —Dissolve components of C in 25—30 ml of distilled water.

IV —Heat separately A, C & D to 50—60°C. First add A to D with constant stirring. After the mixing is complete then add C to this mixture with stirring. Finally add the solution of components of B to the mixture with stirring. Complete the preparation with addition of perfume and remaining water. Homogenise the mixture to get a satisfactory hair dressing.

EMULSION HAIR DRESSING

|  | Ex. 6 | Ex. 7 | Ex. 8 |
|---|---|---|---|
| 1. Mineral oil | 19.50 | 18.00 | 19.50 |
| 2. Isopropyl myristate | 1.00 | 1.00 | 1.00 |
| 3. Cetyl alcohol | 1.20 | — | 1.20 |
| 4. Emulsol TK* | 2.00 | — | 2.00 |
| 5. Arlacel-83* | 1.00 | 1.00 | 1.00 |
| 6. Gelatin | 0.50 | — | 0.50 |
| 7. Polyvinyl pyrollidone | — | 2.00 | — |
| 8. Glycerol | — | 5.00 | — |
| 9. Stearic acid | — | 2.00 | — |
| 10. Lanolin | — | 1.50 | — |
| 11. Histidine | 2.00 | 2.00 | 4.00 |

4

## 0 008 171

### EMULSION HAIR DRESSING (con'd.)

| | Ex. 6 | Ex. 7 | Ex. 8 |
|---|---|---|---|
| 12. Methionine | 0.50 | 0.50 | 0.50 |
| 13. p-Aminobenzoic acid | 1.50 | 1.00 | 0.8 |
| 14. Folic acid | 1.00 | 0.50 | 0.50 |
| 15. Inositol | 1.00 | 1.50 | 1.00 |
| 16. Magnesium sulphate | 0.20 | 0.20 | 0.20 |
| 17. Zinc sulphate | 0.40 | 0.40 | 0.40 |
| 18. Egg lecithin | 0.50 | 0.50 | 0.50 |
| 19. NaOH | to pH 6.5—7.5 | to pH 6.5—7.5 | to pH 6.5—7.5 |
| 20. Methyl p-hydroxy benzoate | 0.10 | 0.10 | 0.10 |
| 21. Perfume | 0.50 | 0.50 | 0.50 |
| 22. Distilled water | to 100 | to 100 | to 100 |

\* Trade Mark

Method of preparation of pourable emulsion hair dressing

I —Oil phase:
Heat ingredients 1, 2, 3, 4, 5, 9, 10, 18, 20, 21 to 50—60°C.

II —Aqueous phase A:
Make a slurry in water of ingredients 6, 11, 12, 13, 14, 15 and to this add sufficient amount of NaOH to bring the pH to 6.5—7.5 when a clear solution will be formed. Heat the solution to 50—60°C.

III —Aqueous phase B:
Dissolve ingredients 7, 8, 16 and 17 in a small amount of water and heat to 50—60°C.

First mix the oil phase and aqueous phase A with vigorous stirring. To this mixture add aqueous phase B and homogenise several times.

### ALCOHOL BASED LOTION

| | Example 9 | Example 10 |
|---|---|---|
| 1. Isopropyl myristate | 2.0 | 1.0 |
| 2. Tween 20* | 1.0 | 1.0 |
| 3. Alcohol | 15.0 | 10.0 |
| 4. Isopropyl alcohol | — | 5.0 |
| 5. Polyvinyl pyrrolidone | 0.5 | 0.5 |
| 6. Bronidiol* | 0.1 | 0.1 |
| 7. Histidine | 2.0 | 2.5 |

5

ALCOHOL BASED LOTION (cont'd.)

|  | Example 9 | Example 10 |
|---|---|---|
| 8. PABA | 1.0 | 0.9 |
| 9. Methionine | 0.1 | 1.0 |
| 10. Folic acid | 0.3 | 0.2 |
| 11. Inositol | 0.5 | 0.5 |
| 12. NaOH | to pH 6.5—7.5 | to pH 6.5—7.5 |
| 13. Perfume | 0.5 | 0.5 |
| 14. Distilled water | to 100 | to 100 |

\* Trade Mark

Method of preparation of lotion

Prepare an aqueous slurry of ingredients 5 to 11. To this add sufficient 20% aqueous solution of NaOH to bring pH to 6.5—7.5 thus obtaining a clear solution. To this solution add an aqueous alcoholic solution containing ingredients 1, 2, 4 and perfume to produce an alcoholic lotion.

The compositions as described in each of Examples 1 to 10 will significantly increase or improve hair growth or hair regrowth when applied to the hair and scalp of male subjects showing male pattern baldness.

Example 11

This example describes the invention in terms of a hair tonic formulation which was applied during the course of a clinical trial to the hair and scalp of male human subjects who exhibited varying degrees of baldness.

Hair tonic formulation

The hair tonic formulation employed in the clinical trial was a coconut oil based hair cream which contained the following ingredients:

| | % w/w |
|---|---|
| Coconut oil | 40.000 |
| Bees wax | 2.300 |
| Stearic acid | 2.225 |
| Cetyl alcohol | 0.200 |
| Lanolin | 0.300 |
| Nipagin* | 0.100 |
| Butyl hydroxyanisole | 0.100 |
| Egg lecithin | 0.500 |
| Arlacel-83* | 0.200 |
| Magnesium sulphate, $7H_2O$ | 0.510 |
| Zinc sulphate | 0.440 |
| L-Histidine monohydrochloride | 5.000 |
| DL-Methionine | 0.500 |
| P-Aminobenzoic acid | 1.000 |
| Folic acid | 0.500 |
| Sodium hydroxide | 1.000 |
| Perfume | 0.500 |
| Distilled water | 30.000 |
| Lime water | 14.625 |
| | 100.000 |

\* Trade Mark

Subjects

22 male subjects took part in the clinical trial. Each subject either had marked frontal baldness with a noticeable bald patch on the scalp, or showed only receding hairline typical of the onset of male pattern baldness.

Test design

Each subject was asked to collect daily the hairs lost by combing. The basal daily hair loss value was determined for each subject for 30 days before the start of the trial with the hair tonic. The subjects were then given a tube containing about 20 g hair tonic cream, and instructed to apply it once or twice a day with massage. Each subject was required to recover the hair lost daily by combing and a number of hairs lost before and after use of the cream was recorded.

Hair diameter

In the case of 16 subjects, the hair diameter was determined on the hairs collected before use and 6 months after use of the hair tonic cream. The hair diameter was measured from the hair bulb at 1 mm intervals up to a distance of about 10 mm. It was observed that from 4 to 6 mm from the hair bulb along the hair shaft, the diameter was essentially constant. Hair diameter was accordingly measured at 6 mm from the hair bulb and an average value for the hair diameter was calculated in the case of each subject.

Results

From an observation of the median daily hair loss of all the subjects both before and after use of the hair tonic, a general decrease in hair loss became apparent after 3 months' use of the hair tonic. The results were analysed statistically and it was shown that there was a significant reduction in daily hair loss up to the end of the trial which was, 12 months from the first application of the hair tonic.

The head of such subject was photographed at intervals during the trial, and new hair growth was observed in the bald patches of most of those subjects who had bald patches at the commencement of the trial.

Of the 16 subjects whose hair diameter had been measured during the course of the trial, a statistically significant increase in hair diameter was observed after application of the hair tonic.

Conclusion

The results of the clinical trial showed that regular use of the hair tonic cream according to the invention reduced hair loss, stimulated new growth and tended to increase hair diameter. These effects were noticeable after 3 months' application of the hair tonic cream and further improvements were noted up to 12 months which marked the conclusion of the trial.

**Claims**

1. A hair and scalp treatment composition comprising
   (a) from 0.1 to 5% by weight of a diazole chosen from imidazole, and histidine and histidine monohydrochloride, and mixtures thereof;
   (b) from 0.1 to 5% by weight of a monobasic acid chosen from methionine, p-aminobenzoic acid and mixtures thereof;
   (c) from 0.1 to 5% by weight of folic acid; and
   (d) a physiologically acceptable carrier.

2. A composition according to claim 1, in which the diazole is imidazole and forms from 1 to 3% by weight of the composition.

3. A composition according to claim 1 or 2, which comprises histidine, methionine and p-aminobenzoic acid.

4. A composition according to any preceding claim, in which the diazole is histidine and forms from 0.2 to 3% by weight of the composition.

5. A composition according to any preceding claim, in which the diazole is 1-histidine monohydrochloride.

6. A composition according to claim 1, 2 or 3, in which the monobasic acid is methionine and forms from 0.2 to 3% by weight of the composition.

7. A composition according to claim 1, 2, 3 or 6, in which the methionine is dl-methionine.

8. A composition according to claim 1, 2 or 3, in which the monobasic acid is p-aminobenzoic acid and forms from 0.2 to 3% by weight of the composition.

9. A composition according to any preceding claim, in which folic acid forms from 0.2 to 2% by weight of the composition.

10. A method of treating human hair which comprises applying to the hair and/or scalp a composition according to any preceding claim.

**Patentansprüche**

1. Haar- und Kopfhaut-Behandlungsmittel mit
   (a) 0,1 bis 5 Gew.-% eines Diazols, ausgewählt unter Imidazol und Histidin und Histidin-Monohydrochlorid und deren Gemischen;
   (b) 0,1 bis 5 Gew.-% einer einbasigen Säure, ausgewählt unter Methionin, p-Aminobenzoesäure und deren Gemischen;
   (c) 0,1 bis 5 Gew.-% Folsäure und
   (d) einem physiologisch annehmbaren Träger.

2. Mittel nach Anspruch 1, in dem das Diazol Imidazol ist und 1 bis 3 Gew.-% des Mittels bildet.

3. Mittel nach Anspruch 1 oder 2, das Histidin, Methionin und p-Aminobenzoesäure enthält.

4. Mittel nach einem der vorhergehenden Ansprüche, in dem das Diazol Histidin ist und 0,2 bis 3 Gew.-% des Mittels ausmacht.

5. Mittel nach einem der vorhergehenden Ansprüche, in dem das Diazol 1-Histidin-Monohydrochlorid ist.

6. Mittel nach Anspruch 1, 2 oder 3, in dem die einbasige Säure Methionin ist und 0,2 bis 3 Gew.-% des Mittels ausmacht.

7. Mittel nach Anspruch 1, 2, 3 oder 6, in dem das Methionin dl-Methionin ist.

8. Mittel nach Anspruch 1, 2 oder 3, in dem die einbasige Säure p-Aminobenzoesäure ist und 0,2 bis 3 Gew.-% des Mittels ausmacht.

**0 008 171**

9. Mittel nach einem der vorhergehenden Ansprüche, in dem Folsäure 0,2 bis 2 Gew.-% des Mittels ausmacht.

10. Verfahren zur Behandlung menschlichen Haares, bei dem auf das Haar und/oder die Kopfhaut ein Mittel gemäß einem der vorhergehenden Ansprüche aufgebracht wird.

## Revendications

1. Composition de traitement du cheveu et du cuir chevelu comprenant
   (a) de 0,1 à 5% en poids d'un diazole choisi parmi l'imidazole, et l'histidine et le monochlorhydrate d'histidine, et leurs mélanges;
   (b) de 0,1 à 5% en poids d'un acide monobasique choisi parmi le méthionine, l'acide p-aminobenzoïque et leurs mélanges;
   (c) de 0,1 à 5% en poids d'acide folique; et
   (d) un support physiologiquement acceptable.

2. Composition selon la revendication 1, où le diazole est l'imidazole et forme de 1 à 3% en poids de la composition.

3. Composition selon la revendication 1 ou 2, qui comprend l'histidine, la méthionine et l'acide p-aminobenzoïque.

4. Composition selon l'une quelconque des revendications précédentes, où le diazole est l'histidine et forme de 0,1 à 3% en poids de la composition.

5. Composition selon l'une des revendications précédentes, où le diazole est le monochlorhydrate de 1-histidine.

6. Composition selon les revendications 1, 2 ou 3, où l'acide monobasique est la méthionine et forme de 0,1 à 3% en poids de la composition.

7. Composition selon les revendications 1, 2, 3 ou 6, où la méthionine est le dl-méthionine.

8. Composition selon les revendications 1, 2 ou 3, où l'acide monobasique est l'acide p-aminobenzoïque et forme de 0,2 à 3% en poids de la composition.

9. Composition selon l'une des revendications précédentes où l'acide folique forme de 0,2 à 2% en poids de la composition.

10. Procédé de traitement du cheveu humain qui implique d'appliquer aux cheveux et/ou au cuir chevelu une composition selon l'une des revendications précédentes.